# EUROPÄISCHE PATENTSCHRIFT

(11) **EP 1 917 996 B1**
(45) Veröffentlichungstag und Bekanntmachung des Hinweises auf die Patenterteilung: **18.02.2009**
(21) Anmeldenummer: 06022700.6
(22) Anmeldetag: 31.10.2006
(51) Int. Cl.: A61M 39/10

(54) **Nicht wieder entkonnektierbares positiver Luer-Lock Anschlussstück**
Luer Lock connector which is not disconnectable
Connecteur Luer lock positif qui n'est plus détachable

(43) Veröffentlichungstag der Anmeldung: 07.05.2008
(73) Patentinhaber: Codan Holding GmbH, 23738 Lensahn (DE)
(72) Erfinder: Krüger, Peter, 23738 Damlos (DE)
(74) Vertreter: Fleck, Thomas

(56) Entgegenhaltungen:
- WO-A-99/37356
- DE-U1- 20 109 061
- US-A- 6 152 913

## Beschreibung

Die Erfindung betrifft ein Luer-Lock positiv Anschlussstück nach dem Oberbegriff des Anspruches 1, sowie einen Kanülenhalter oder Katheter, der das Luer-Lock positiv Anschlussstück besitzt.

Derartige Luer-Lock positiv (männliche) Anschlussstücke bzw. Adapter nach DIN EN 1707 sind in den unterschiedlichsten Ausführungsformen bekannt. Üblicherweise sind diese wieder lösbar.

Aus der US 6,152,913 ist eine Luer-Lock-Verbindung bekannt, die die Wahrscheinlichkeit des unbeabsichtigten Lösens durch eine erhöhte Reibung bezüglich der Drehbewegung zum Entkonnektieren durch eine spezielle Ausgestaltung von aneinander reibenden konischen Flächen reduziert.

Die DE 201 09 061 beschreibt eine Luer-Lock-Verbindung, mit Drehmoment begrenzter Überlastkupplung zur zuverlässigen Sicherstellung der Entkonnektierbarkeit.

Gerade bei der Verabreichung von toxischen und generell für die Umwelt gefährlichen Medien, wie z.B. Zytostatika, ist es jedoch wünschenswert, jegliches Entkonnektieren der Luer-Lock-Verbindung, ob beabsichtigt oder unbeabsichtigt, sicher zu verhindern, damit weder Patienten, noch Personal mit dem gefährlichen Medium in Berührung kommen, welches durch gelöste Verbindungen austreten kann. Insofern besteht also Bedarf an einem positiven Luer-Lock Adapter bzw. Anschlussstück, das ein beabsichtigtes oder unbeabsichtigtes Entkonnektieren der Verbindung wirksam vermeidet und jegliche Gefährdungssituation ausschließt.

Diese Aufgabe wird erfindungsgemäß dadurch gelöst bzw. das Problem dadurch beseitigt, dass es eine mit dem Lock-Gewinde wirksam verbundene Verzahnung aufweist und dass eine Überwurfhülse unlösbar und um den Schaft drehbar auf diesen aufgesetzt ist, die in ihrem Inneren eine passgerecht zu der Verzahnung ausgebildete Gegenverzahnung aufweist, wobei die Verzahnung und die Gegenverzahnung so ausgebildet sind, dass sie in einer ersten Drehrichtung zum Verriegeln der Luer-Lock-Verbindung der Überwurfhülse auf dem Schaft arretierend ineinander greifen, in einer zweiten, der ersten Drehrichtung entgegen gesetzten Drehrichtung jedoch freilaufend aneinander abgleiten, und dass an dem rückwärtigen Schaft (23) und/oder der Überwurfhülse (20) Rastmittel (16) zum unlösbaren Verrasten der Überwurfhülse (20) auf dem rückwärtigen Schaft (23) angeordnet sind.

Erfindungsgemäß gewährleistet die spezielle geometrische Kontur des Anschlussstückes, dass eine sichere flüssigkeits- und/oder gasdichte Verbindung permanent vorhanden bleibt und eine Entkonnektierung ausgeschlossen werden kann. Die erfindungsgemäß vorgesehene Verzahnung am Lock-Gewinde und die Gegenverzahnung im Inneren der Überwurfhülse ermöglichen ein Festziehen des Anschlussstücks durch beispielsweise Rechtsdrehung, wobei ein Losdrehen in entgegen gesetzter Drehrichtung durch Freilauf von Verzahnung und Gegenverzahnung verhindert wird.

Die Erfindung richtet sich ebenfalls auf einen Kanülenhalter oder Katheter, der das erfindungsgemäß ausgebildete Luer-Lock positiv Anschlussstück besitzt.

Weitere Vorteile und Merkmale gehen aus den Unteransprüchen 2 bis 6 hervor, die auch gemeinsam mit dem Hauptanspruch von erfinderischer Bedeutung sein können.

Im Folgenden werden zwei bevorzugte Ausführungsbeispiele anhand der Figuren näher erläutert, auf die die Erfindung jedoch nicht beschränkt ist. Sie dienen lediglich dem besseren Verständnis der Erfindung und ihrer Funktionen. Es zeigt:
- Fig. 1: eine Seitenansicht eines erfindungsgemäßen, zusammengesetzten Kanülenhalters mit Schutzkappe;
- Fig. 2: eine schematische, dreiteilige Querschnittsansicht in Form einer Explosionsdarstellung der Einzelbestandteile des in Fig. 1 gezeigten erfindungsgemäßen Kanülenhalters;
- Fig. 3: Seitenansichten zu den in Fig. 2 gezeigten Querschnittsansichten der Einzelbestandteile;
- Fig. 4: eine Seitenansicht einer weiteren Ausführungsform eines erfindungsgemäßen, zusammengesetzten Kanülenhalters mit Schutzkappe;
- Fig. 5: schematische Querschnittsansichten in Explosionsform des in Fig. 4 gezeigten Kanülenhalters mit seinen Einzelbestandteilen ; und
- Fig. 6: Seitenansichten der in Fig. 5 gezeigten Einzelbestandteile des Kanülenhalters.

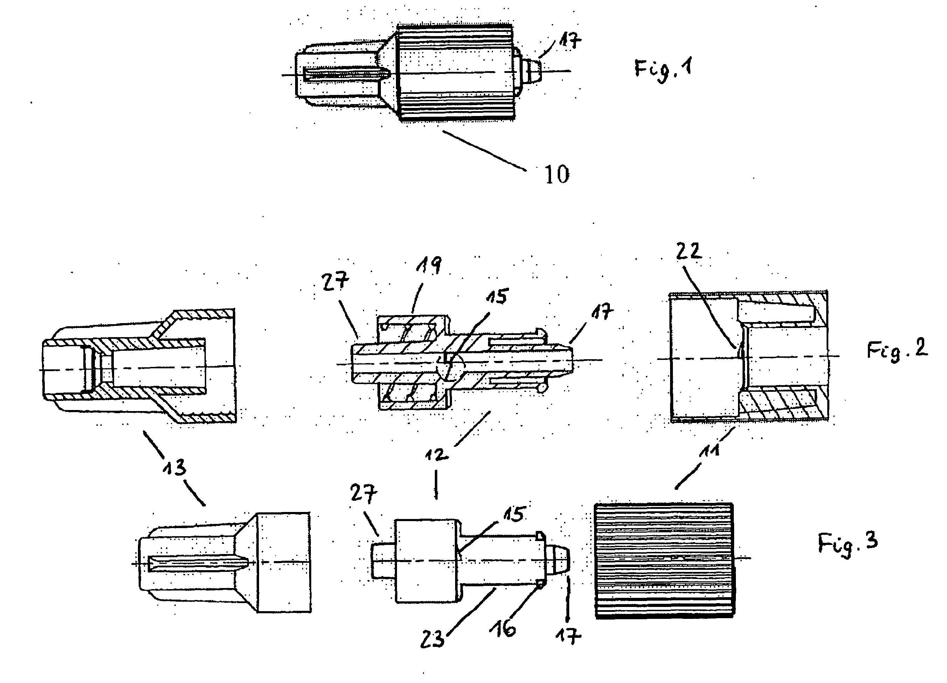

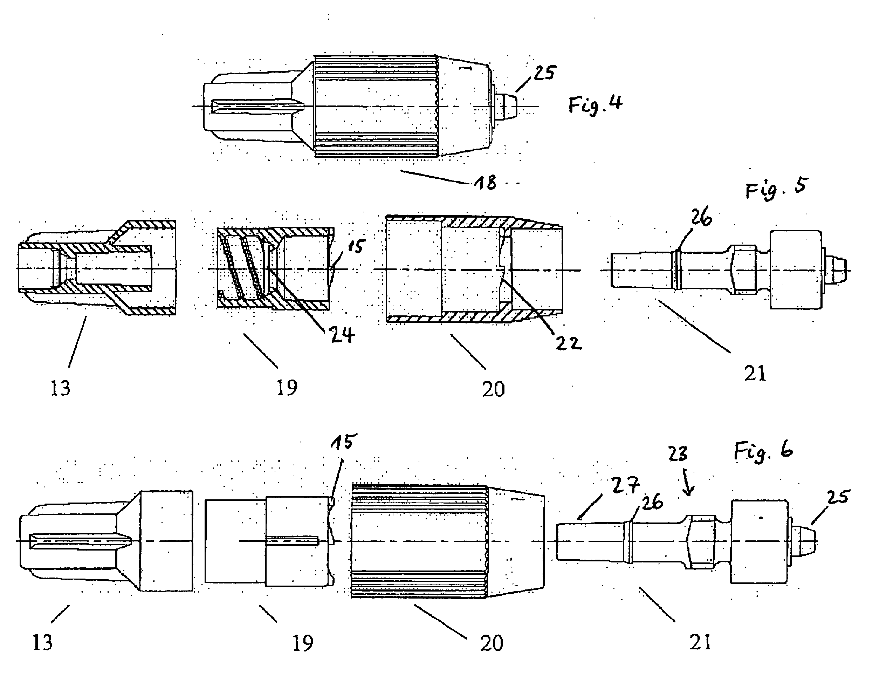

In den Figuren 1 und 4 ist jeweils eine Ausführungsform eines erfindungsgemäßen Kanülenhalters gezeigt, der mit 10 bzw. 18 bezeichnet ist und seine endgültige nicht wieder entkonnektierbare Form besitzt. Während die erste Ausführungsform mit Schutzkappe 13 dreiteilig ist, besteht die zweite Ausführungsform des erfindungsgemäßen Kanülenhalters mit Schutzkappe 13 aus vier Teilen. Die Schutzkappe 13 ist von keiner besonderen erfindungsgemäßen Bedeutung. Am rechtsseitigen Ende befindet sich der Anschluss 17 bzw. 25 für einen nicht gezeigten Schlauch. Das linksseitige Ende der Kanülenhalter 10 bzw. 18 wird durch die Schutzkappe 13 gebildet, welche als Flow-Stop fungiert. In etwa mittig ist die geriffelte Überwurfhülse 11 bzw. 20 angeordnet. Die Größenverhältnisse der Einzelbestandteile, die nachfolgend näher erläutert werden, sind üblich, ebenso wie das Kunststoffmaterial, das beispielsweise aus ABS, Polycarbonat, Polyethylen oder Polyamid ausgewählt werden kann. Andere Materialien sind für den Fachmann denkbar.

Nachfolgend soll auf den besonderen Aufbau des Luer-Lock positiv Anschlussstücks 12 bzw. Luer Anschlussstückes 21 anhand der Figuren 2 und 3 bzw. 5 und 6 eingegangen werden.

Von besonderer erfindungsgemäßer Bedeutung für die erste Ausführungsform, die in den Figuren 2 und 3 gezeigt wird, ist die mit dem Gewinde 19 verbundene Verzahnung 15 im erfindungsgemäßen Luer-Lock Anschluss 12, über den eine Überwurfhülse oder -mutter 11 aufgesetzt ist, die um den Schaft drehbar ist, jedoch von letzterem nicht abgezogen werden kann. Eine zur Verzahnung 15 passgerechte Gegenverzahnung 22 ist im Inneren der außen geriffelt gestalteten Überwurfhülse 11 vorgesehen. Die Verzahnungen 15 und 22 greifen in einer ersten Drehrichtung arretierend ineinander, während sie in entgegen gesetzter Drehrichtung freilaufend aneinander abgleiten. Letzteres wird über kontinuierlich und kantenlos schräg verlaufende Flanken bewerkstelligt, die in eine Grundfläche übergehen. Die Überwurfhülse 11 kann vom rückwärtigen Schaft 23 nicht mehr abgezogen werden, da ein umlaufender Rastvorsprung 16 in seinem Endbereich vorgesehen ist.

Unter Bezugnahme auf die Figuren 5 und 6 wird nunmehr eine zweite Ausführungsform der Erfindung näher erläutert. Während in der ersten Ausführungsform das Lock-Gewinde 19 fest mit dem Luer-Konus 27 und dem rückwärtigen Schaft 23 verbunden und einstückig damit ausgebildet ist, ist in der zweiten Ausführungsform das Lock-Gewinde 19 in einer separaten Ausgestaltung vorgesehen und auf den rückwärtigen Schaft 23 des Luer-Lock positiv Anschlussstückes 21 frei drehbar aufgesetzt. Auch in dieser Ausführungsform wirken die Verzahnung 15 am Lockgewinde 19 mit der Gegenverzahnung 22 in der Überwurfmutter 20 auf die gleiche Art und Weise zusammen wie es bei der ersten Ausführungsform der Fall war. Die Arretierung des Luer positiv Anschlussstücks 21 erfolgt über einen Schnappverschluss, bei dem ein ringartiger Vorsprung 26 auf dem Schaft 23 mit einer passgerechten Ausnehmung 24 zusammenwirkt, die in etwa mittig umlaufend im Lock-Gewinde 19 vorgesehen ist. Der Schnappverschluss garantiert, dass das Lock-Gewinde 19 nach Montage auf dem Anschlussstück 21 nicht mehr von diesem abgezogen werden kann. Dadurch wird verhindert, dass der Kanülenhalter 18 wieder entkonnektiert werden kann, weil das drehbare Lock-Gewinde mittels Schnappverschluss unlösbar mit dem Anschlussstück 21 verbunden ist. Ein Vorteil gegenüber der ersten Ausführungsform ist darin zu sehen, dass sich das Anschlussstück 21 während des Anziehens nicht mitdreht, wodurch verhindert wird, dass sich der nicht gezeigte Übertragungsschlauch beim Anschluss des Gerätes in axialer Richtung verdreht und es zu einer möglichen Abknickung desselben und damit zu einer Patientengefährdung kommen kann.

## Patentansprüche

1. Luer-Lock positiv Anschlussstück zum Anschluss an alle normgerechten negative Luer-Lock Anschlüsse mit einem Luer-Konus (27), einem sich an den Luer-Konus (27) anschließenden, rückwärtigen Schaft (23) und einem Lock-Gewinde (19), einer mit dem Lock-Gewinde (19) wirksam verbundenen Verzahnung (15) und einer unlösbar und um den Schaft (23) drehbar aufgesetzten Überwurfhülse (11,20), die in ihrem Inneren eine passgerecht zu der Verzahnung (15) ausgebildete Gegenverzahnung (22) aufweist, dass die Verzahnung (15) und die Gegenverzahnung (22) so ausgebildet sind, dass sie in einer ersten Drehrichtung zum Verriegeln der Luer-Lock-Verbindung der Überwurfhülse (11,20) auf dem Schaft (23) arretierend ineinander greifen, in einer zweiten, der ersten Drehrichtung entgegen gesetzten Drehrichtung jedoch freilaufend aneinander abgleiten, **dadurch gekennzeichnet dass** an dem rückwärtigen Schaft (23) und/oder der Überwurfhülse (20) Rastmittel (16) zum unlösbaren Verrasten der Überwurfhülse (20) auf dem rückwärtigen Schaft (23) angeordnet sind.

2. Luer-Lock positiv Anschlussstück nach Anspruch 1, **dadurch gekennzeichnet, dass** die Verzahnung (15) und die Gegenverzahnung (22) entgegengesetzt gerichtete Angriffsflächen aufweisen, die rückwärtig jeweils über kontinuierlich und kantenlos schräg verlaufende Flanken in eine Grundfläche übergehen.

3. Luer-Lock positiv Anschlussstück nach Anspruch 1, **dadurch gekennzeichnet, dass** die Verzahnung (15) auf einer quer zu einer Längsachse des rückwärtigen Schaftes (23) rückwärtig zu der Verzahnung (15) liegenden Fläche und die Gegenverzahnung (22) auf einer innerhalb der Überwurfhülse (11,20) gelegenen Stirnfläche angebracht sind.

4. Luer-Lock positiv Anschlussstück nach einem der Ansprüche 1 bis 3, **dadurch gekennzeichnet, dass** das Lock-Gewinde (19) fest mit dem Luer-Konus (27) und/oder dem rückwärtigen Schaft (23) verbunden, vorzugsweise einstückig damit ausgebildet, ist.

5. Luer-Lock positiv Anschlussstück nach einem der Ansprüche 1 bis 3, **dadurch gekennzeichnet, dass** das Lock-Gewinde (19) in einer separaten auf den rückwärtigen Schaft (23) frei um diesen drehbar aufgesetzten Überwurfhülse (20) ausgebildet ist.

6. Luer-Lock positiv Anschlussstück nach einem der vorstehenden Ansprüche, **dadurch gekennzeichnet, dass** es aus Kunststoff, insbesondere ABS oder Polycarbonat, besteht.

7. Kanülenhalter oder Katheter zur Verwendung bei der Verabreichung von Zytostatika oder anderen toxischen und generell für die Umwelt gefährlichen Medien an Patienten mit einem Luer-Lock positiv Anschlussstück (12,21) nach einem der vorstehenden Ansprüche an seinem einen Ende und mit Anschluss (17,25) für einen Übertragungsschlauch an seinem anderen Ende.

8. Kanülenhalter oder Katheter nach Anspruch 7, **dadurch gekennzeichnet, dass** er eine auf den Luer-Konus (27) des Luer-Lock positiv Anschlussstückes (12,21) aufgesetzte Schutzkappe (13) aufweist.

## Claims

1. Positive Luer Lock connector for connecting to all standard-conforming, negative Luer Lock connections with a Luer cone (27), a rear shank (23) connected to the Luer cone (27) and a lock thread (19), a toothing (15) actively connected to the lock thread (19) and a bush (11, 20) which is non-detachable and mounted in rotary manner about the shank (23) and which has in its interior a countertoothing (22) constructed in precise fitting manner with respect to the toothing (15), the toothing (15) and countertoothing (22) being so constructed that in a first rotation direction they engage in one another in arresting manner for locking the Luer Lock connection of the bush (11, 20) on shank (23) and in a second rotation direction opposite to the first rotation direction they slide in free-running manner on one another, **characterized in that** on the rear shank (23) and/or the bush (20) are provided detent means (16) for the non-detachable fixing of the bush (20) to the rear shank (23).

2. Positive Luer Lock connector according to claim 1, **characterized in that** the toothing (25) and countertoothing (22) have oppositely directed contact surfaces, which pass rearwardly in each case via continuously and edgeless sloping flanks into a base surface.

3. Positive Luer Lock connector according to claim 1, **characterized in that** the toothing (15) is applied to a surface positioned transversely to a longitudinal axis of the rear shank (23) and rearwards with respect to the toothing (15) and the countertoothing (22) is applied to an end face located within the bush (11, 20).

4. Positive Luer Lock connector according to one of the claims 1 to 3, **characterized in that** the lock thread (19) is firmly connected to the Luer cone (27) and/or the rear shank (23) and is preferably integrally constructed therewith.

5. Positive Luer Lock connector according to one of the claims 1 to 3, **characterized in that** the lock thread (19) is formed in a separate bush (20) mounted on the rear shank (23) so as to be freely rotatable about the same.

6. Positive Luer Lock connector according to one of the preceding claims, **characterized in that** it is made from plastic, particularly ABS or polycarbonate.

7. Cannula holder or catheter for use in the administration of cytostatics or other toxic media which are generally harmful to the environment to patients with a positive Luer Lock connector (12, 21) according to one of the preceding claims at its one end and with a connection (17, 25) for a transfer hose at its other end.

8. Cannula holder or catheter according to claim 7, **characterized in that** it has a protective cap (13) mounted on the Luer cone (27) of the positive Luer Lock connector (12, 21).

## Revendications

1. Elément de raccordement Luer-Lock positif pour le raccordement à tous les raccords Luer-Lock négatifs normalisés avec un cône Luer (27), une tige (23) arrière raccordée au cône Luer (27) et un filet de verrouillage (19), une denture (15) raccordée de manière active au filet de verrouillage (19) et une douille d'accouplement (11, 20) posée de manière indétachable et rotative autour de la tige (23), qui comporte à l'intérieur une denture antagoniste (22) conçue pour s'ajuster à la denture (15), la denture (15) et la denture antagoniste (22) étant conçues de telle sorte que, dans un premier sens de rotation, elles viennent en prise l'une dans l'autre en formant un blocage pour le verrouillage du raccordement Luer-Lock de la douille d'accouplement (11, 20) sur la tige (23) alors que, dans un second sens de rotation opposé au premier, elles glissent librement l'une à côté de l'autre, **caractérisé en ce que** des éléments encliquetables (16) sont montés sur la tige arrière (23) et / ou sur la douille d'accouplement (20) pour l'encliquetage indétachable de la douille d'accouplement (20) sur la tige arrière (23).

2. Elément de raccordement Luer-Lock positif selon la revendication 1, **caractérisé en ce que** la denture (15) et la denture antagoniste (22) comportent des surfaces de prise dirigées à l'opposé l'une de l'autre qui se prolongent chacune vers l'arrière par une surface de base par l'intermédiaire de flancs s'étendant en biais de manière continue et sans arêtes.

3. Elément de raccordement Luer-Lock positif selon la revendication 1, **caractérisé en ce que** la denture (15) est montée sur une surface posée à l'arrière de la denture (15) perpendiculairement à un axe longitudinal de la tige arrière (23) et la denture antagoniste (22) est posée sur une face frontale située à l'intérieur de la douille d'accouplement (11, 20).

4. Elément de raccordement Luer-Lock positif selon l'une quelconque des revendications 1 à 3, **caractérisé en ce que** le filet de verrouillage (19) est raccordé fixement au cône Luer (27) et / ou à la tige arrière (23), de préférence réalisé d'un seul tenant avec celui-ci et / ou celle-ci.

5. Elément de raccordement Luer-Lock positif selon l'une quelconque des revendications 1 à 3, **caractérisé en ce que** le filet de verrouillage (19) est formé dans une douille d'accouplement (20) séparée posée sur la tige arrière (23) de manière à pouvoir tourner librement autour de celle-ci.

6. Elément de raccordement Luer-Lock positif selon l'une quelconque des revendications précédentes, **caractérisé en ce qu'**il est constitué de matière plastique, en particulier d'ABS ou de polycarbonate.

7. Porte-canule ou cathéter destiné à être utilisé pour l'administration de cytostatiques ou d'autres substances toxiques et généralement dangereuses pour l'environnement à des patients à l'aide d'un élément de raccordement Luer-Lock positif (12, 21) selon l'une quelconque des revendications précédentes à l'une de ses extrémités et avec un raccord (17, 25) pour un flexible de transmission à son autre extrémité.

8. Porte-canule ou cathéter selon la revendication 7, **caractérisé en ce qu'**il comporte un capot de protection (13) posé sur le cône Luer (27) de l'élément de raccordement Luer-Lock positif (12, 21).
